Europäisches Patentamt

European Patent Office

Office européen des brevets

(11) Publication number: **0 324 261**
**A1**

## EUROPEAN PATENT APPLICATION

(21) Application number: 88312161.8

(22) Date of filing: 22.12.88

(51) Int. Cl.⁴: **C07D 307/62**

(30) Priority: 06.01.88 US 142098

(43) Date of publication of application:
**19.07.89 Bulletin 89/29**

(84) Designated Contracting States:
**AT BE CH DE ES FR GB GR IT LI LU NL SE**

(71) Applicant: PFIZER INC.
235 East 42nd Street
New York, N.Y. 10017(US)

(72) Inventor: Lozanov, Merrill
13 Willow Lane
East Lyme Connecticut(US)

(74) Representative: Wood, David John et al
Pfizer Limited Ramsgate Road
Sandwich Kent CT13 9NJ(GB)

(54) Production of L-ascorbic acid from 2-keto-L-gulonic acid with an acid catalyst and a lipophilic surfactant having a low hydrophilic: lipophilic balance under substantially anhydrous conditions.

(57) A method for producing L-ascorbic acid which comprises (a) forming a substantially anhydrous slurry of 2-keto-l-gulonic acid and at least one lipophilic surfactant having a low hydrophilic:lipophilic balance of from about 4.3 to about 10.2 in a supporting organic layer which optionally contains a co-solvent and (b) reacting said slurry with a substantially anhydrous hydrogen chloride gas acid catalyst to convert said 2-keto-l-gulonic acid to L-ascorbic acid.

EP 0 324 261 A1

# PRODUCTION OF L-ASCORBIC ACID FROM 2-KETO-L-GULONIC ACID WITH AN ACID CATALYST AND A LIPOPHILIC SURFACTANT HAVING A LOW HYDROPHILIC:LIPOPHILIC BALANCE UNDER SUBSTANTIALLY ANHYDROUS CONDITIONS

This invention relates to the production of L-ascorbic acid from 2-keto-l-gulonic acid, and, more particularly, to the production of L-ascorbic acid from 2-keto-l-gulonic acid with a lipophilic surfactant having a low hydrophilic:lipophilic balance under substantially anhydrous conditions with substantially anhydrous hydrogen chloride gas acid catalyst.

The most successful and common method of producing L-ascorbic acid is based on a multi-step synthesis from d-glucose going through sorbose and 2-keto-l-gulonic acid as described by Reichstein and Grussner, Helv. Chim. Acta., 17, 311-328 (1934). L-ascorbic acid is obtained by heating 2-keto-l-gulonic acid in water at 100°, or by esterifying 2-keto-l-glulonic acid (L-xylo-2-hexulosonic acid), which is in turn converted into L-ascorbic acid by treatment with sodium methoxide in methanol, followed by acidification with hydrogen chloride gas.

A summary of the various techniques for producing L-ascorbic acid is found in Advances in Carbohydrate Chemistry and Biochemistry, Vol. 37, pp. 79-155 (1980). L-ascorbic acid, aside from its use as a vitamin (Vitamin C), has numerous other industrial and commercial uses.

A Japanese patent publication relating to the production of L-ascorbic acid, 48(1973) - 15931, based on application number 87,371, filed October 5, 1970, is reported in Chemical Abstracts, Vol. 79, 1973, p. 352 (53169z). This patent publication discloses the preparation of L-ascorbic acid by treating diacetone-2-keto-l-2-keto-l-gulonic acid with concentrated hydrochloric acid in the presence of inactive solvents and surfactants. By way of example, this publication discloses heating 100 grams of diacetone-2-keto-1-gulonic acid, 300 ml of benzene, 0.3 grams of stearylpropylenediamine dioleate, and 10 ml of concentrated hydrochloric acid for five hours at 65° C to give 58.7 grams of L-ascorbic acid (purity 97.5%).

Patent Cooperation Treaty International Application W087/00839, published February 12, 1987, which claims priority from U.S. patent application serial no. 764,262, filed August 9, 1985, describes the single step conversion of 2-keto-l-gulonic acid to L-ascorbic acid under substantially anhydrous conditions with a surfactant and substantially anhydrous hydrogen chloride acid gas catalyst.

None of these references describe or suggest conducting the single step conversion of 2-keto-l-gulonic acid (hereinafter sometimes referred to as 2-KGA) to L-ascorbic acid with a lipophilic surfactant having a low hydrophilic:lipophilic balance of from about 4.3 to about 10.2 under substantially anhydrous conditions, or that conducting such a reaction under such conditions produces both high yield and purity such that the crude L-ascorbic acid can be efficiently converted to finished goods L-ascorbic acid.

Broadly stated, the present invention contemplates a method for producing L-ascorbic acid by (a) forming a substantially anhydrous slurry of 2-keto-l-gulonic acid and a lipophilic surfactant having a low hydrophilic:lipophilic balance of from about 4.3 to about 10.2 in a supporting organic layer which optionally contains a co-solvent, and (b) reacting said slurry with a substantially anhydrous hydrogen-chloride gas acid catalyst to convert said 2-keto-l-gulonic acid to L-ascorbic acid. A preferred method for producing L-ascorbic acid utilizes a hydrate of 2-keto-l-gulonic acid, a sorbitan fatty acid ester surfactant and an alcohol co-solvent.

The general method for producing L-ascorbic acid comprises (a) forming a substantially anhydrous slurry (i.e., without addition of free water beyond that present in small amounts due to processing conditions and that present as water of hydration in the defined ketone) of 2-keto-l-gulonic acid and a lipophilic surfactant having a low hydrophilic:lipophilic balance of from about 4.3 to about 10.2 in a supporting organic layer optionally containing a co-solvent, and (b) reacting said slurry with a substantially anhydrous acid catalyst to convert said 2-keto-l-gulonic acid to L-ascorbic acid. When utilizing a hydrate of 2-keto-l-gulonic acid (defined below) as a reactant, the one-step reaction of the present invention proceeds well without addition of free water beyond that present in very small amounts due to processing conditions. Increasing the amount of free water above such amount decreases yield. This undesirable effect is due in part to the decomposition of the L-ascorbic acid which takes place in the presence of an aqueous acid.

The 2-keto-l-gulonic acid intermediate can be obtained by a number of well known synthetic routes starting from commercially available L-sorbose. Protection of L-sorbose as a diacetonide can be accomplished in acetone using dimethyoxypropane with a sulfuric acid catalyst. The diacetone-l-sorbose can then be oxidized using basic sodium hypochlorite in the presence of a nickel chloride catalyst to give diacetone-2-ketogulonic acid. Removal of the protecting group of the diacetone-2-ketogulonic acid can be accomplished by heating it in distilled water to produce 2-keto-l-gulonic acid.

A convenient method for achieving substantially anhydrous reaction conditions is through the use of a hydrate of 2-keto-l-gulonic acid. While 2-keto-l-gulonic acid $1H_2O$ is a true hydrate, further water may be associated with the solid as unbound water. Drying 2-keto-l-gulonic acid in a vacuum at 50°C overnight gives 2-keto-l-gulonic acid $nH_2O$, where n is a hydrate from about 0 to about 0.5. Air-drying overnight at ambient temperature gives 2-keto-l-gulonic acid $nH_2O$, where n is a hydrate from 1 to about 2. A preferred material is produced by drying overnight at ambient temperatures to yield a hydrate of 2-keto-l-gulonic acid $nH_2O$ wherein n is about 1.5. There appears to be a correlation between the hydration state of 2-keto-l-gulonic acid and its conversion of L-ascorbic acid. Generally, as the hydration state of 2-keto-l-gulonic acid decreases below some optimal level, there is a corresponding decrease in the yield of its conversion to L-ascorbic acid. This can be controlled by the amount of water in the 2-keto-l-gulonic acid, hydrogen chloride, or co-solvent, and is presumably required to dissolve hydrogen chloride in the reaction mixture. It should be noted that the dependence on hydration state is observed under substantially anhydrous (i.e., water starved) conditions. Very small amounts of water can be added to the reaction medium to aid in cyclization; however, no additional water need be added if the hydration state is sufficiently high (e.g., at least about 0.1 and, preferably, about 1.5), providing other reaction criteria are met.

L-ascorbic acid is a white crystalline solid melting at 192° and having, in water, a specific rotation at the sodium D line of +24°. In solution, L-ascorbic acid has a $pK_1$ of 4.17 and a $pK_2$ of 11.79.

L-ascorbic acid is known to decompose in aqueous acids via a first order reaction with a half life of 5 hours at 70°C. Therefore, minimizing the time that L-ascorbic acid is in contact with aqueous hydrochloric acid is critical. This is accomplished in the present invention by slurrying the 2-keto-l-gulonic acid in a supporting organic layer which serves as the reaction medium. The supporting organic layer can be an aromatic hydrocarbon solvent, an aliphatic hydrocarbon solvent, a halogenated hydrocarbon solvent or a mixture thereof. Representative aromatic solvents include toluene, xylene, benzene, ethyl benzene and mixtures thereof. A preferred organic solvent is toluene, either alone or in combination with other organic or aliphatic solvents. Representative aliphatic solvents include hexane, heptane, octane, nonane, decane, dodecane and mixtures thereof. Representative halogenated solvents are dichloromethane, chloroform, carbon tetrachloride and dichloroethane.

Nonionic, cationic and anionic surfactants can be used in this invention; however, nonionic and cationic surfactants, including mixtures thereof, are preferred. More preferred nonionic and cationic surfactants are those having a low hydrophilic:lipophilic balance (HLB). Surfactants with a HLB of from about 4.3 to about 10.2 will operate effectively with ≤1 equivalent of water. Optimum water content is dependent on surfactant type, with less water being required with the more lipophilic surfactants. It should also be noted that surfactants having lower HLB will produce a significant improvement in the color of the crude L-ascorbic acid, because, in part, of the capacity of the more lipophilic surfactants to solubilize in the organic phase more of the color impurities which form during the reaction. Lighter colored crudes, having less color insolubles and fewer impurities, are easier to purify. Examples of surfactants which can be used in the present invention include quaternary ammonium salts, phosphonium salts, amines (which are cationic sources), crown ethers, ethoxylated alcohols, cryptands, aminopolyethers, phosphorylsulfoxides, certain naturally occurring ionophores, glycerol esters, sorbitan fatty acid esters and mixtures thereof. While a particular surfactant may have a HLB in excess of 10.2 or less than 4.3, the present invention can utilize such a surfactant as long as it is used with another surfactant such that the average HLB of the two surfactants is from about 4.3 to about 10.2. Representative surfactants include hexadecyltrimethylammonium chloride, oleylamine, tetrabutylammonium chloride, benzyltriethylammonium chloride, trioctylmethylammonium chloride, tricaprylmethylammonium chloride, tetrabutylammonium hydrogen sulfate, dodecyltrimethyl ammonium chloride, octadecyltrimethyl ammonium chloride, di(hydrogenated tallow)-dimethyl ammonium chloride, polyoxyethylene(15)-octadecylmethyl ammonium chloride, octadecylbis(2-hydroxyethyl)methyl ammonium chloride, oleylbis(2-hydroxyethyl)methyl ammonium chloride, polyethoxylated amine, oleylbis (2-hydroxyethyl)amine, sorbitan monolaurate, sorbitan tristearate, sorbitan monoleate, and mixtures thereof. More preferred surfactants are those which do not contain nitrogen. The sorbitan fatty acid esters are a most preferred class of surfactants. Typical amounts of surfactants are from 1 mg to about 250 mg for every gram of 2-keto-l-gulonic acid.

Those skilled in the art will recognize other surfactant materials that are useful in connection with the reaction of the invention. A general listing of surfactants can be found in McCutcheon's Emulsifier & Detergents I, 1983 McCutcheon Pub. Co.

This anhydrous or substantially anhydrous acid catalyst can be one or more mineral acids. Representative mineral acids include hydrochloric, sulfuric, phosphoric and hydrofluoric. A preferred mineral acid is hydrogen chloride gas. The advantages of substantially anhydrous hydrogen chloride gas over other acids are cost, ease of removal from the L-ascorbic acid, low side product formation relative to other mineral acids, and shorter reaction times. In reactions where minute quantities of water are present, such as when the rate of hydration of 2-keto-l-gulonic acid serves as the aqueous phase, only enough substantially anhydrous hydrogen chloride gas to saturate this water may be necessary to produce L-ascorbic acid in high yield.

A co-solvent can be utilized to increase the solubility of the gaseous, anhydrous hydrogen chloride acid catalyst. The co-solvent can be alcohols, ethers, esters or ketones, or a mixture thereof. Examples of co-solvents that can be used in the present invention include methanol, ethanol, propanol, isopropanol, butanol, hexanol, heptanol, octanol, acetone, isopropyl ether, and mixtures thereof. A preferred class of co-solvent is alcohol, particularly hexanol and octanol. The use of a co-solvent effectively reduces overall consumption of substantially anhydrous, hydrogen chloride gas acid catalyst consumption by a factor of 10 or more over a system not containing a co-solvent. The conversion of 2-keto-l-gulonic acid to L-ascorbic acid in the presence of a co-solvent proceeds normally; however, some adjustment may have to be made in the HLB of the surfactant to prevent the solids from aggregating towards the end of the reaction. Typical amounts of co-solvent are from about 0.01 ml to about 0.04 ml for every gram of 2-keto-l-gulonic acid.

The yield and the purity of the L-ascorbic acid are also sensitive to the time and temperature of reaction. The optimal reaction temperature is in the range of from about 40°C to about 80°C. The optimal time of reaction is in the range of from about 2 hours to about 5 hours. In general, lower reaction temperatures give poorer conversions requiring longer reaction times. In general, higher temperatures give acceptable conversions but lower crude yields (decomposition may occur). A preferred reaction time is from about 2 hours to about 4 hours. A preferred reaction temperature is from about 60°C to about 70°C. A reaction time of about 3 hours at a temperature of about 65°C produces high crude yields of L-ascorbic acid. The reaction can, of course, be conducted at atmospheric or superatmospheric pressure.

"Crude Yield" is the amount of total solids recovered from the reaction expressed as a percent of the stoichiometric amount of product. "Yield corrected for purity" represents the amounts of pure product contained in the total solids and is numerically equal to the crude yield times the assay of the product. Assay by $I_2$ titre, expressed in percent purity, indicates the amount of ascorbic acid in the crude product, and is determined by standard iodimetry using Thyodene indicator (product of Magnus Chemical Co.). Crystallization yield represents the amount of purified product recovered by crystallization of the crude product, and is expressed as a percent of product (corrected for purity) introduced into the crystallization step. Accountability indicates the total amount of product by assay obtained after crystallization, including both isolated ascorbic acid and the product material in the mother liquor.

Refining of the crude entails dissolution of the solids in hot (60°C) water, decolorization by activated carbon, filtration of the solution over filter aid followed by a hot water wash; the solution is then concentrated, cooled with slow agitation in a water/ice bath, and the solids allowed to crystallize for 1 to 2 hours, then filtered. The crop is washed with acetone (or methanol, ethanol, or isopropanol), and dried. The wash is combined with the mother liquor and either multiple crops taken successively after concentration of each mother liquor, or the mother liquor is recycled in the recovery scheme. The recovered crops are combined and tested for finished good quality. The quality control specifications include (but are not limited to):

Appearance - white to slight yellow crystalline granules essentially free of foreign matter

Odor - none

Assay - 99.0 to 100.5%

Specification - (+)20.5° to (+)21.5°
[10% w/v aq., 25°C, D]

Melt range - 189 to 192°C

Solution color:
[30 g/90 ml H₂O]

% transmission - min 90%

Color reflectance - max + 4.5

Clarity - essentialy clear with not more than trace of fibers and foreign matter

Heavy metals - max 10 ppm

Since the reaction of the invention proceeds best under substantially anhydrous conditions, it is carried out by including the 2-keto-l-gulonic acid(hydrate) in a slurry and bubbling hydrogen chloride gas through the slurry. Aqueous HCl is not used because it decomposes L-ascorbic acid. However, small amounts of concentrated HCl can be used to facilitate the reaction. The materials used to form the slurry and thus aiding in promoting the reactions of the invention may vary substantially depending on factors such as the intended end use for the L-ascorbic acid (e.g., industrial, pharmaceutical, etc.). Accordingly, different materials may be used depending on economic and purity requirements. Depending on the ultimate application of the L-ascorbic acid, it will, of course, be necessary to use materials (e.g., solvents,

4

surfactants, reaction intermediates, etc.) which comply with various state, federal or foreign regulations.

The aforesaid invention is illustrated by the following specific examples, some of which (e.g. Examples 1 through 5) are presented to show results with surfactants that have hydrophilic:lipophilic balances outside of the range of from about 4.3 to about 10.2.

EXAMPLE 1

Into a 25 ml. 3-neck flask is placed 20 ml of toluene and 50 mg of hexadecyltrimethylammonium chloride (HLB 15.8). The solution is heated to 65°C and 5.0 gms, 0.023 moles of 2-keto-l-gulonic acid (1.5 hydrate) are added all at once which forms a slurry. Hydrogen chloride gas is bubbled in at a rate of 80 ml/min. for 3 hours. The toluene is then removed by rotary evaporation under reduced pressure at 30°C. The solid residue is diluted with 20 ml of toluene and again evaporated to ensure all water is removed. The solid is washed 2 times with toluene and dried under vacuum overnight. The yield is 4.18 gms, 0.0226 moles, 99%, of the tan, 1/2 hydrate of L-ascorbic acid. m.p. = 184-187°C, 93/0 wt% L-ascorbic acid/2-keto-l-gulonic acid (by high pressure liquid chromatography) [a]$_D$ = +47.8° (c = 1 in methanol).

EXAMPLE 2

Into a 25 ml, 3-neck flask is placed 20 ml of toluene and 50 mg of hexadecyltrimethylammonium chloride (HLB 15.8). The solution is heated to 65°C and 5.0 gms of 2-keto-l-gulonic acid (1.5 hydrate) are added, followed immediately by 1.0 ml of concentrated HCl. Following this, HCl gas is blown through the slurry at a rate of 80 ml/minute for 2.5 hours. The toluene is removed at 30°C under reduced pressure. This step is repeated twice to ensure dryness. The light brown solid is dried in vacuum overnight. The yield is 4.32 gms., 0.0213 moles, 94% m.p. = 180-184°C; 90/1 wt% L-ascorbic acid/2-keto-l-gulonic acid (by high pressure liquid chromatography).

EXAMPLE 3

Into a 25 ml, 3-neck flask is placed 20 ml of toluene, 1 ml of distilled water and 50 mg of hexadecyltrimethylammonium chloride (HLB 15.8). The mixture is heated to 65°C and 5.0 gms of 2-keto-l-gulonic acid (1.5 hydrate) are added all at once. With stirring, hydrogen chloride gas is bubbled through the solution for 5 hours. After cooling to 30°C, toluene is removed by rotary evaporation at reduced pressure. After adding additional toluene and removing it two additional times, the tan solid is dried. The yield is 5.32 gms, 0.0213 moles, 94%, m.p. = 181-185°C; 85/1 wt% L-ascorbic acid/2-keto-l-gulonic acid (by high pressure liquid chromatography).

EXAMPLE 4

Into a 25 ml, 3-neck RB flask equipped with stirrer, thermometer, heating mantle, and sparger are placed 20 ml toluene and 41.2 mg dodecyltrimethylammonium chloride (HLB 17.1). The mixture was heated to 65°C with stirring, and 5.44g 2-KGA·1.48H$_2$O (24.1 mmoles) were added all at once. Temperature was maintained at 65°C, and anhydrous HCl gas was bubbled through the reaction medium at 80 cc/min. Reaction was carried out until no 2KGA was detected by HPLC (about 3 hours), at which point reaction was stopped, the mixture cooled to R.T., then stripped to dryness by rotary evaporation. To the solids, 10 to 20 ml toluene were added and stripped again. The solids were then washed with 2 x 10 ml toluene. On vacuum drying, 4.12 gm of greyish tan product was obtained (23.4 mmoles, 97.1%). The product assayed 92.8% by I$_2$ titre. The yield corrected for purity was 90.1%.

EXAMPLE 5

Following the procedure of EXAMPLE 4, the reaction was conducted with 50 mg hexadecyltrimethyl ammonium chloride (HLB 15.8) and 22.7 mmoles 2-KGA·1.48H$_2$O. On vacuum drying, 3.87 gm of greyish tan product was obtained (22.0 mmoles). The product assayed 93.3% by I$_2$, titre. The yield corrected for purity was 90.5%.

## EXAMPLE 6

Following the procedure of EXAMPLE 4, the reaction was conducted with 30 mg di(hydrogenated tallow)dimethyl ammonium chloride (HLB 9.7) and 23.4 mmoles 2-KGA·1.25H$_2$O. On vacuum drying,4.05 gm of light grey product were obtained (23.0 mmoles). The product assayed 94.5% by I$_2$ titre. The yield corrected for purity was 92.9%.

## EXAMPLE 7

Following the procedure of EXAMPLE 4, the reaction was conducted with 55 mg oleylbis(2-hydroxyethyl)amine (HLB 9.9) and 23.0 mmoles 2-KGA·1.5H$_2$O. On vacuum drying, 3.85 gm of medium greyish tan product was obtained (21.9 mmoles). The product assayed 89.9% by I$_2$ titre. The yield corrected for purity was 85.6%.

## EXAMPLE 8

Following the procedure of EXAMPLE 4, the reaction was conducted with 56 mg sorbitan monolaurate (HLB 8.6) and 18 mmoles 2-KGA·1.26H$_2$O. On vacuum drying, 4.22 gm of light grey product were obtained (24.0 mmoles). The product assayed 94.7% by I$_2$ titre. The yield corrected for purity was 91.4%.

## EXAMPLE 9

Following the procedure of EXAMPLE 4, the reaction was conducted with 56 mg sorbitan monolaurate (HLB 8.6) and 18 mmoles 2-KGA 1.08H$_2$O. On vacuum drying, 3.78 gm of light grey product was obtained (21.5 mmoles). The product assayed 95.0% by I$_2$ titre. The yield corrected for purity was 91.6%.

## EXAMPLE 10

Following the procedure of EXAMPLE 4, the reaction was conducted with 50 mg sorbitan monooleate (HLB 4.3) and 23.0 mmoles 2-KGA 1.5H$_2$O. On vacuum drying 3.98 gm of light grey product was obtained (22.6 mmoles). The product assayed 93.9% by I$_2$ titre. The yield corrected for purity was 92.3%.

## EXAMPLE 11

Following the procedure of EXAMPLE 4, the reaction was conducted with 105 mg sorbitan monolaurate (HLB 8.6) and 34 mmoles 2-KGA 0.5H$_2$O. On vacuum drying 5.83 gm of light grey product was obtained (33.15 mmoles). The product assayed 95.3% by I$_2$ titre. The yield corrected for purity was 92.9%.

## EXAMPLE 12

Following the procedure of EXAMPLE 4, the reaction was conducted with 70 mg sorbitan monolaurate (HLB 8.6) and 22.5 mmoles 2-KGA 0.8$H_2O$. On vacuum drying, 3.83 gm of light grey product was obtained (21.76 mmoles). The product assayed 96.2% by $I_2$ titre. The yield corrected for purity was 913.0%.

## EXAMPLE 13

Following the procedure of EXAMPLE 4, the reaction was conducted with 56 mg sorbitan monolaurate (HLB 8.6) and 18 mmoles 2-KGA·1.08$H_2O$. On vacuum drying, 3.05 gm of light grey product was obtained (17.35 mmole). The product assayed 91.6% by $I_2$ titre. The yield corrected for purity was 91.6%.

## EXAMPLE 14

Following the procedure of EXAMPLE 4, the reaction was conducted with 52 mg sorbitan monolaurate (HLB 8.6) and 23.3 mmole 2-KGA·1.26$H_2O$. On vacuum drying, 4.22 gm of light grey product was obtained (23.97 mmole). The product assayed 94.7% by $I_2$ titre. The yield corrected for purity was 91.4%.

## EXAMPLE 15

Following the procedure of EXAMPLE 4, the reaction was conducted with 70 mg sorbitan monolaurate (HLB 8.6) and 23.0 mmole 2-KGA·1.38$H_2O$. On vacuum drying, 3.92 gm of medium grey product was obtained (22.26 mmole). The product assayed 93.9% by $I_2$ titre. The yield corrected for purity was 90.9%.

## EXAMPLE 16

### Recrystallization of Crude Ascorbic Acid

Crude solids were dissolved in water at 60°C (3cc water per gram of crude), and stirred under a nitrogen blanket. Nuchar SA (Westvac Chem.), 2 parts per 100 parts of ascorbic acid (as determined by assay of the crude) was added and stirring continued for 30 minutes. The mixture was filtered over SuperCel (Johns-Manville), washed with 60°C water (1cc per gm crude) and the filtrate plus wash concentrated to 1/2 volume. The concentrate was cooled to 0-5°C with slow agitation under a nitrogen blanket, and allowed to crystallize 1 hour (with seeding if necessary), then filtered. The crop was washed with acetone, 2cc per gm crystals (est. weight), and the wash combined with the mother liquor. Multiple crops were taken, successively after concentration of each mother liquor. In each case, the crop was washed with acetone and the wash combined with the mother liquor before concentration. This refining treatment gave the results indicated in the following table.

7

| Surfactant | HLB | Ascorbic Acid (Combine Crops) % | Ascorbic Acid Remaining in Mother Liquor % | Accountability % | Combined Crops | |
|---|---|---|---|---|---|---|
| | | | | | Assay, % | Color |
| BTMAC[1] | 15.8 | 90.0 | 6.0 | 96.0 | 94.2 | greyish white |
| ODTMAC[2] | 15.7 | 87.9 | 7.7 | 95.6 | 93.8 | tanish white |
| OHEMAC[3] | 10 | 91.0 | 4.9 | 95.9 | 92.2 | light tan |
| OHEA[4] | 9.9 | 91.5 | 5.2 | 96.7 | 99.3 | white with tinge off yellow |
| DTMAC[5] | 9.7 | 91.2 | 5.3 | 96.5 | 99.0 | white with tinge off yellow |
| Span 20[6] | 8.6 | 91.9 | 4.8 | 96.7 | 99.0 | sl. off white |
| Span 80[7] | 4.3 | 93.6 | 3.4 | 97.0 | 98.7 | sl. off white |

1 - hexadecyltrimethyl ammonium chloride
2 - octadecyltrimethyl ammonium chloride
3 - oleybis (2-hydroxyethyl)methyl ammonium chloride
4 - oleybis (2-hydroxyethyl)amine
5 - di(hydrogenated tallow)dimethyl ammonium chloride
6 - sorbitan monolaurate
7 - sorbitan monooleate

## EXAMPLE 17

### Refining of Crude Ascorbic Acid

Crude ascorbic acid product, 44.8 grams, from reaction using sorbitan monolaurate was dissolved in 100 cc water at 55°-60°C under a nitrogen blanket. Nuchar SA, 0.7 gm was added with moderate agitation, and followed by 5.0 gm of SuperCel. The mixture was stirred for 30 minutes, filtered hot (60°C) on a Buchner funnel over SuperCel precoat with a cross stream of nitrogen over the surface. The filter cake was washed with 25 cc of 60°C water (maintaining nitrogen stream). The filtrate was then concentrated by rotary evaporator to 60 cc, at 55°C, then transferred to a beaker with 10 cc water rinse. The concentrate was cooled to 0-5°C under a nitrogen blanket and granulated for 1.5 hours. The grain was filtered, washed with 15 cc cold methanol, then vacuum dried at 40°C to give 27.68 gms of white, crystalline product with an assay of 99.9% by $I_2$ titre. The 1st mother liquor plus wash was concentrated to 22 cc, 7 cc methanol was added, the solution cooled to 0.5°C, and granulated for 1.5 hours under a nitrogen blanket. The grain was filtered, washed with 10 cc cold methanol, vacuum dried to yield 8.33 gm of white, crystalline product, 99.4% by $I_2$ titre. The 2nd mother liquor was concentrated to 10 cc, 3 cc of methanol was added, and the solution cooled to 0-5°C under a nitrogen blanket and granulated 1 hour. The grain was filtered, washed with 5 cc cold methanol and vacuum dried to yield 5.18 gm of product with a slight tinge of color and an assay of 98.6% by $I_2$ titre. The 3rd mother liquor plus wash was taken to dryness to give 3.3 grams of solids, eggshell in color, which assayed 81.8% by $I_2$ titre.

## EXAMPLE 18

### Quality Control Testing of Refined Ascorbic Acid

The refining procedure of EXAMPLE 17 was repeated two more times. The first crop from EXAMPLE 17 was combined with the first crop from each of the two repeated experiments, and the combined crop is identified below as Crop 1. The same procedure was followed for the second and third crops, and the combined crops are identified below as Crop 2 and Crop 3, respectively. The quality control analysis of the three crops is indicated in the following table.

|  |  | Appearance | Assay | Specific Rotation | Melt Range | Reflectance Color |
|---|---|---|---|---|---|---|
| Specifications |  | White to slightly Yellow Crystals | 99-100.5% | (+)20.0 to (+21.5) | 189-192°C | Max +4.5 |
| Crop 1 | meets test |  | 100.1% | (+)21.1° | 189-190° | 2.20 |
| | 2 | " |  | 99.9% | (+)21.1° | 189-190° | 2.41 |
| | 3 | " |  | 98.8% | (+)21.1° | 186.5-187.5° | 2.49 |

## EXAMPLE 19

### Cyclization of 2-keto-l-gulonic Acid With Mixed Co-Solvent

In 100 cc 3 neck RB flask equipped with stirrer, thermometer, heating mantle, and gas sparger was placed 52.5 cc toluene and 0.18 cc octanol (99%), then 0.105 gram of sorbitan monolaurate and 0.025 gram of hexadecyltrimethyl ammonium chloride was added. The mixture was heated to 65°C with stirring, and 12.75 gm 2KGA·0.9H$_2$O (0.06 mole) was added all at once; temperature was maintained at 65°C, and ca 1.41 anhy. HCl gas was bubbled in intermittently. The system was closed between additions of HCl gas, and reaction was carried out until no 2KGA was detected by HPLC (3.1 hours).

The reaction was stopped, cooled, the solvent was filtered off, and the solids repulped with 17 ml toluene plus 3 ml acetone, then filtered. Residual water in the solids was azeotroped with 20 ml toluene on the rotary evaporator at 45°C. On vacuum drying (45°C), 10.45 gm (98.8% crude yld) of light grey product was obtained. Assays by I$_2$ titre was 93.9% and the corrected yield was 92.8%.

## EXAMPLE 20

Following the procedure of EXAMPLE 19, the reaction was conducted with ethanol (0.5% v/v), 0.18 mole 2KGA·1.08H$_2$O and 1.0 gm sorbitan monolaurate. On vacuum drying, 31.30 gm (97.9% crude yield) of light to medium grey product was obtained. The product assayed 95.0% by I$_2$ titre. The yield corrected for purity was 93.0%.

## EXAMPLE 21

Following the procedure of EXAMPLE 19, the reaction was conducted with ethanol (0.19% v/v), 0.06 mole 2-KGA·0.6H$_2$O and 0.35 gm sorbitan monolaurate. On vacuum drying, 10.22 gm (96.8% crude yield) of light grey product was obtained. The product assayed 96.1% by I$_2$ titre. The yield corrected for purity was 93.0%.

## EXAMPLE 22

Following the procedure of EXAMPLE 19, the reaction was conducted with ethanol (0.19% v/v), 0.06 mole 2-KGA·6H$_2$O, 0.35 gm oleylbis (2-hydroxyethyl)amine and 0.20 gm sorbitan monolaurate. On vacuum drying, 9.83 gm (93.1% crude yield) of light to medium grey product was obtained. The product assayed 96.9% by I$_2$ titre. The yield corrected for purity was 90.2%.

## EXAMPLE 23

Following the procedure of EXAMPLE 19, the reaction was conducted with hexanol (0.34% v/v), 0.06 mole 2-KGA·0.9H$_2$O, 0.105 gm sorbitan monolaurate and 0.025 gm hexadecyltrimethyl ammonium chloride (HLB 15.8). On vacuum drying, 10.48 gm (99.2% crude yield) of light grey product was obtained. The product assayed 93.3% by I$_2$ titre. The yield corrected for purity was 93.8%.

## EXAMPLE 24

Purification of L-ascorbic acid

Into a 50 ml. 3-neck roundbottom flask is placed 20 ml of distilled water. The crude ascorbic acid, 2.0 gms is dissolved into the water at room temperature and 0.2 gms of decolorizing charcoal is added. The slurry is filtered through diatomaceous earth and the filter cake washed with 5 ml of water. The water is removed by rotary evaporation and the white solid is washed with toluene and dried in a vacuum oven. The yield is 1.90 gms of L-ascorbic acid; m.p. = 180-181°C.

The purification procedure used varies depending on factors such as the materials used to form the slurry (e.g., toxic, non-toxic), the desired end use of the L-ascorbic acid (e.g., industrial, pharmaceutical) availability of materials, time and economics.

The present invention has been disclosed and described herein with the inclusion of specific embodiments. However, the scope of the invention is limited only by the claims and not specific embodiments.

## Claims

1. A method for producing L-ascorbic acid comprising:

(a) forming a substantially anhydrous slurry of 2-keto-l-gulonic acid and at least one lipophilic surfactant having a low hydrophilic:lipophilic balance of from about 4.3 to about 10.2 in a supporting organic layer

(b) reacting said slurry with substantially anhydrous, hydrogen chloride gas acid catalyst to convert said 2-keto-l-gulonic acid to L-ascorbic acid.

2. A method according to claim 1 , wherein said lipophilic surfactant is selected from the group consisting of sorbitan fatty acid esters, hexadecyltrimethyl ammonium chloride, dodecyltrimethyl ammonium chloride, octadecyltrimethyl ammonium chloride, di (hydrogenated tallow)dimethyl ammonium chloride, polyoxyethylene(15)octadecylmethyl ammonium chloride, octadecylbis(2-hyroxyethyl)methyl ammonium chloride, oleylbis(2-hydroxyethyl)methyl ammonium chloride, polyethoxylated amine, oleylbis(2-hydroxyethyl)amine, and mixtures thereof.

3. A method according to claim 2 , wherein said lipophilic surfactant is selected from the group consisting of sorbitan monolaurate, oleylbis(2-hydroxyethyl)amine, di(hydrogenated tallow)dimethyl ammonium chloride, sorbitan monooleate and mixtures thereof.

4. A method according to claim 2 , wherein said supporting organic layer contains a co-solvent.

5. A method according to claim 4 , wherein said co-solvent is selected from the group consisting of methanol, ethanol, propanol, isopropanol, butanol, hexanol, heptanol, octanol and mixtures thereof.

6. A method according to claim 1 , wherein said lipophilic surfactant is selected from the group consisting of sorbitan monolaurate, sorbitan tristearate, sorbitan monooleate, cationic surfactants and mixtures thereof; and, wherein said co-solvent is selected from the group consisting of ethanol, isopropanol, butanol, hexanol, octanol and mixture thereof.

7. A method according to claim 6 , wherein said 2-keto-l-gulonic acid is hydrate.

8. A method according to claim 7 , wherein said hydrate is 2-keto-l-gulonic acid $\cdot nH_2O$ wherein n is from about 0.1 to about 2.0.

9. A method according to claim 6 , wherein said supporting layer is selected from the group consisting of toluene, xylene, hexane, heptane, octane, nonane, decane and mixtures thereof.

10. A method according to claim 1 , wherein said aromatic solvent is toluene, said surfactant is selected from the group consisting of sorbitan fatty acid esters and mixtures thereof, and said co-solvent is selected from the group consisting of alcohols and mixtures thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | WO-A-8 700 839 (THE LUBRIZOL CORPORATION) <br> * the whole document * <br> --- | 1 | C 07 D 307/62 |
| A,D | CHEMICAL ABSTRACTS <br> vol. 79, no. 9, 3rd September 1973, page 359, Columbus, Ohio, US, column 2, abstract no 53169z; & JP - A - 73 15931 (TAKED A CHEM. IND.) 18-05-1973 <br> --- | 1 | |
| A | CHEMICAL ABSTRACTS <br> vol. 106, no. 23, 8th June 1987, page 777, column 1, Columbus, Ohio, US, abstract no. 196736u; & CZECH. CS 234886, 15-01-1987 <br> --- | 1 | |
| A,P | EP-A-0 264 649 (AIR PRODUCTS AND CHEMICALS) <br> * page 12, claim 11 * <br> ----- | 1 | |

TECHNICAL FIELDS SEARCHED (Int. Cl.4)

C 07 D 307/00

The present search report has been drawn up for all claims

| Place of search | Date of completion of the search | Examiner |
|---|---|---|
| BERLIN | 28-03-1989 | KYRIAKAKOU G |